# EUROPEAN PATENT APPLICATION

(11) **EP 1 930 016 A1**
(43) Date of publication of application: **11.06.2008**
(21) Application number: 06025252.5
(22) Date of filing: 06.12.2006
(51) Int. Cl.: A61K 35/12

(54) **Use of Langerhans cells for the induction of immune tolerance**

(71) Applicant: Bernhard-Nocht-Institut für Tropenmedizin, 20359 Hamburg (DE)
(72) Inventor: The designation of the inventor has not yet been filed
(74) Representative: Vossius & Partner

(57) **Abstract**

The present invention relates to the use of epidermal Langerhans cells (LCs) transfected with a nucleic acid molecule encoding, in expressible form, an antigen or comprising an externally introduced antigen for the preparation of a pharmaceutical composition for inducing immune tolerance in a subject. Furthermore, the invention relates to the use of particles suitable for particle bombardment and an antigen or a nucleic acid molecule encoding an antigen for the preparation of a pharmaceutical composition for inducing immune tolerance in a subject. In addition, the invention relates to the use of an antigen for the preparation of a composition for inducing immune tolerance in a subject, wherein said antigen is to be applied to the skin of a subject prior to particle bombardment of said skin, wherein said bombardment is suitable to introduce said antigen to a LC in the epidermis.

## Description

The present invention relates to the use of epidermal Langerhans cells (LCs) transfected with a nucleic acid molecule encoding, in expressible form, an antigen or comprising an externally introduced antigen for the preparation of a pharmaceutical composition for inducing immune tolerance in a subject. Furthermore, the invention relates to the use of particles suitable for particle bombardment and an antigen or a nucleic acid molecule encoding an antigen for the preparation of a pharmaceutical composition for inducing immune tolerance in a subject. In addition, the invention relates to the use of an antigen for the preparation of a composition for inducing immune tolerance in a subject, wherein said antigen is to be applied to the skin of a subject prior to particle bombardment of said skin, wherein said bombardment is suitable to introduce said antigen to a LC in the epidermis.

In this specification, a number of documents are cited. The disclosure content of these documents including manufacturers' manuals is herewith incorporated by reference in its entirety.

Immunotherapy has gained wide acceptance as a promising measure to address several disease states including autoimmune diseases, transplant rejection, allergies, infectious diseases and cancer. Despite rapid and exciting progress in approaches to treatment, the disease burden attributable to such illnesses has not significantly abated. The complex nature of the normal and pathologic immunological processes associated with such diseases, coupled with problems in evaluating and implementing methods for immunotherapy in human subjects, continue to be some of the obstacles to successful advances in treatment. Successful approaches to immunotherapy are based on the ability of the immunotherapeutic molecule to be delivered in a therapeutic, sub-toxic dose at the desired therapeutic frequency. In the process of selection of a suitable therapeutic molecule, it is recognized that sub-toxic doses may be insufficient for the desired therapeutic effect, especially when the antibody binds incidentally to cell populations other than the target population. In the case of an injectable preparation and especially an intravenous mode of delivery, in contrast to readily self-administered modes of delivery, the optimal dosing frequency for therapeutic purposes could impose an undesirable burden on the patient and care-giver, assuming that such optimal frequency to begin with is deemed convenient for clinical trials.

Allergies and autoimmune diseases, e. g. multiple sclerosis, rheumatoid arthritis or lupus erythomatosis display a class of very cost intensive and difficult to treat diseases. Together with undesired immune responses elicited upon transplantation of foreign organs they form the class of diseases caused by a misguided immune system.

Transplantation of allogeneic donor cells, tissues or organs (transplantation between genetically different individuals of the same species) is used to treat a variety of conditions (typically tissue, or organ-failure conditions) and is often the sole or highly preferred therapeutic option. The list of successfully transplanted cells, tissues and organs includes kidney, heart, lung, liver, corneas, pancreas, marrow, skin, and bones. However, allogeneic transplantation involves significant risks and drawbacks, including graft rejection, complications from immunosuppressive therapy and graft versus host disease or host versus graft disease which are frequently highly debilitating or lethal. Rejection of allografts is presently understood to be initiated by the recognition of allogeneic (i. e. donor) major histocompatibility complex (MHC) molecules by recipient T-cells, leading to upregulated cellular and humoral immunity through activation of T-cells. The MHC antigens are typically presented to the recipient T-cells by antigen presenting cells, such as macrophages and dendritic cells. Although immunosuppressive drugs such as cyclosporine may be used in an attempt to modulate rejection, these immunosuppressive agents have severe side effects and often fail to completely prevent rejection. Upon reception of an allogenic graft the receiver is forced to adhere to lifelong medication to prevent rejection of the new organ due to a different set of transplantation antigens.

Immunosuppressive strategies continue to evolve, with a number of new formulations having been developed in recent years. Although acute rejection rates may have diminished, current protocols of immunosuppression for chronic organ rejection are clearly inadequate. This complication remains the primary cause of graft loss months to years after solid organ transplant. In summary, the overall goal of achieving immune tolerance remains elusive.

T-cells expressing CD4 are also known as helper T (Th) cells, and these are regarded as being the most prolific cytokine producers. Cytokines are the hormonal messengers responsible for most of the biological effects in the immune system, such as cell mediated immunity and allergic type responses. This T-cell subset can be further subdivided into Th1 and Th2, and the cytokines they produce are known as Th1-type cytokines and Th2-type cytokines.
Th1-type cytokines tend to produce the proinflammatory responses responsible for killing intracellular parasites and for perpetuating autoimmune responses. Interferon gamma (IFN-γ) is the main Th1 cytokine. Excessive proinflammatory responses can lead to uncontrolled tissue damage, so there needs to be a mechanism to counteract this. The Th2-type cytokines include interleukins 4, 5, and 13, which are associated with the promotion of IgE and eosinophilic responses in atopy, and also interleukin (IL)-10, which has more of an anti-inflammatory response. In excess, Th2 responses will counteract the Th1 mediated microbicidal action. The optimal scenario would therefore seem to be that humans should produce a well balanced Th1 and Th2 response, suited to the immune challenge.

Allergy is a hypersensitivity reaction initiated by immunologic mechanisms as defined by the EAACI nomenclature task force (Johansson et al., 2001). Allergies result when the immune system reacts towards harmless antigens (allergens) that normally are tolerated. Typical allergic symptoms are asthma, rhinitis, conjunctivitis, eczema and gastrointestinal reactions.
During the last decades, the prevalence of allergic diseases has increased dramatically in the industrialised high-income countries. Although more recent studies report stabilising or even decreasing rates, allergic diseases are common.

Both genetic and environmental factors have been suggested to influence the development of allergic diseases.
The allergic reaction involves the production of IgE antibodies to allergens. Both allergic and non-allergic individuals produce allergen specific antibodies but the dominating isotypes differ. Thus non-allergic individuals produce mainly antibodies of the IgG isotype whereas the allergic individuals also produce antibodies of the IgE isotype (Jackola et al., 2002).
Allergic diseases are associated with Th2-like immunity to allergens in affected tissues (Robinson et al., 1992; Mazzarella et al. 2000). The Th2-associated cytokine IL-4 promotes B cell isotype switch to IgE and recruits basophils, eosinophils and monocytes. IL-5 promotes eosinophil cell survival and activation (Gleich, 2000). IL-9 and IL-13 are also involved in the allergic response promoting mucus secretion, airway inflammation, airway hyperresponsiveness and tissue fibrosis (Hauber et al., 2000; Wills-Karp, 2000).
Besides the pro-inflammatory actions of mast cells, basophils, eosinophils and Th2 cells, impaired suppression of the inflammatory responses mediated by regulatory T-cells (Treg) cells has recently been suggested to contribute to the allergic inflammation.
Since allergy is widely regarded as a Th2 weighted imbalance, recently immunologists have been investigating ways to redirect allergic Th2 responses in favour of Th1 responses to try to reduce the incidence of atopy, the personal or familiar tendency to produce IgE antibodies. Some groups have been looking at using high dose exposure to allergen to drive up the Th1 response in established disease, and other groups have been studying the use of mycobacterial vaccines in an attempt to drive a stronger Th1 response in early life.

During the development of immune cells, T-cells undergo a strict selection process in order to prevent autoimmune reactions of the body against its own structures, which often results in diseases as multiple sclerosis, systemic lupus erythomatosus (SLE), juvenile diabetes, rheumatoid arthritis (RA) or Sjögren's syndrome. The initiation of an adaptive CD4+ T-cell immune response depends on the recognition of antigenic peptides by major histocompatibility complex (MHC) II molecules on the surface of antigen presenting cells (APCs) by naïve T-cells. Because of their location at areas of antigen exposition, dendritic cells are assumed to be the initiating APCs.
DCs are bone marrow derived cells that interact with T-cells in secondary lymphoid organs such as lymph nodes (LNs) and spleen. The routes of directing DCs to these organs are complex. As the spleen lacks afferent lymphatic vessels, precursors of spleen DC subsets are likely to proceed from the blood. In contrast to the spleen, skin draining LNs are connected to the periphery via skin-draining lymphatic vessels as well as blood vessels. Consequently, DC subsets found in skin-draining LNs in principle can originate from epidermal LCs as well as dermal DCs that migrate via lymphatic vessels, and from blood-derived DCs. In general, murine DCs constitutively express MHC class II molecules and are positive for the CD11c integrin.
Six DC subsets are currently known in the mouse, to be recognized by different sets of markers. Three of them are blood-derived DCs with the capability to enter secondary lymphoid organs via blood vessels. The remaining three DC subsets migrate via lymphatic vessels from the skin to the draining LNs. These skin-derived DCs represent important APCs that connect the cutaneous compartment with the draining LNs. One of these subsets, derived from epidermal LCs is characterized by the expression of Langerin (CD207), a specific marker for LCs. Langerhans cells are discussed to be crucial for antigen uptake and subsequent presentation to naïve T-cells in skin-draining lymph nodes. LCs migrate under steady-state conditions to the LN, this indicates that a permanent low-level influx of LCs to the paracortical area of the LN takes place (Ritter et al., 2004; Kissenspennig et al., 2005; Stoitzner et al., 2005). Mayerova *et al.* demonstrated that LCs are able to present keratinocyte-expressed self-antigen to naïve T-cells (Mayerova et al., 2004), indicating that the cutaneous system might be involved in peripheral tolerance (Woods et al., 2001).

The by far most extensive in vivo examinations regarding the Th1/Th2 concept were carried out with mice infected with the protozoa *Leishmania* (*L*.) *major.*
*Leishmania* parasites comprise obligate intracellular protozoan pathogens that are adapted to infect a variety of mammalian hosts including humans and mice. Depending on the transmitted parasite subspecies, *Leishmania* infections cause different clinical symptoms that range from cutaneous to visceral manifestations (Ritter et al., 1996; Pearson et al., 1983). In the experimental model of leishmaniasis, mice are infected subcutaneously or intradermally with promastigote *L. major* parasites (Ritter et al., 2004; Belkaid et al., 2002). The course of disease predominantly depends on the genetically determined ability of the infected mouse strain to mount a protective T-cell mediated immune response. This protective Th 1 response is characterized by an early IFN-γ production and the expression of inducible nitric oxide synthase by activated macrophages (Sacks et al., 2002; Alexander et al., 2005). Resistant mouse strains, such as C57BU6 (B6.WT), are able to heal the pathogen-induced skin lesion and acquire immunity. In contrast, susceptible BALB/c mice develop an IL-4 dominated Th2-type response and finally succumb to a progressive generalized infection (Sacks et al., 2002). Since 1993 epidermal LCs have been discussed to be crucial for the induction of a protective immune response against *L. major* (Moll et al., 1993). However, sufficient tools to adequately dissect dermal dendritic cells (DCs) from epidermal LCs were limited or not established in the early 90's. The identification of CD207 (Langerin), a LC-specific marker, now allows for discrimination between lymph node (LN) homing LCs and dermal DCs (Valladeau et al., 2002). Recently it was demonstrated that inflammatory dermal CD207⁺/CD8α⁻ DCs migrate to the draining LN to activate *Leishmania*-specific T-cells (Ritter et al., 2004; Misslitz et al., 2004). It is important to mention that during an infection with *L. major* parasites, LCs also migrate to the paracortical area of skindraining LNs. However, in contrast to dermal DCs, LCs do not induce *Leishmania*-specific T-cell proliferation (Ritter et al., 2004).
Presentation of self-antigen is known to be crucial for induction of tolerance in general. A current model suggests that DCs indeed are tolerogenic when they present antigen under steady-state non-inflammatory conditions. However, when stimulated by inflammatory mediators, they mature and induce effector T-cell responses (Steinman & Nussenzweig, 2002; Probst et al., 2003).
Recently, it has been suggested that LCs exhibit regulatory rather than priming functions. Kaplan *et al.* showed that mice deficient for LCs develop an enhanced contact hypersensitivity that might be the consequence of absent regulatory epidermal LCs (Kaplan et al., 2005).
In C57BU6 mice healing of leishmaniasis correlates with activation of Th1-type immune response. Recently, it could be demonstrated that dermal DCs rather than epidermal LCs are responsible for the activation of Th1 cells after infection. Epidermal application of *Leishmania-*antigen (L-Ag) prior to infection resulted in an atypical course of disease that is characterized by an impaired *Leishmania*-specific Th1 response demonstrating the activation of immunomodulatory effects by epidermal incorporation of *Leishmania*-antigen (Weiss et al., 2006). Consequently, these mice cannot manage an efficient elimination of the parasites at the site of infection.
As described above, LCs can be detected i) under steady-state and ii) under inflammatory conditions in the skin-draining LNs. The experimental approach used included establishment of a method that allows to incorporate L-Ag predominantly into the epidermal compartment of the skin.
The mammalian skin represents an important barrier that separates the external environment from the internal compartments of the organism. The *stratum corneum* is the outermost layer of the epidermis that is in permanent contact with microbes and their associated foreign antigens. This layer consists of dead keratinocytes, a lipohilic matrix, and bactericidal enzymes such as psoriasin (Glaser et al., 2005). In contrast, the epidermal compartment represents a renewing epithelium, comprised of living keratinocytes and a minority of leucocytes, such as LCs and T-cell subsets (Kissenpfennig et al., 2005). Langerin-positive epidermal LCs are known to present L-Ag *in vitro* (Moll et al., 1996). However, it was shown *in vivo* that dermal DCs are responsible for the induction of an antigen-specific immune response (Ritter et al, 2004; Misslitz et al., 2004). This is in line with the established "*LC-paradigm*", postulating that epidermal cells such as LCs might be crucial for the induction of a T-cell immune response.

Weiss et al. (2006) showed that epidermal incorporation of L-Ag modified the Th1-type immune response against intracellular pathogens. Upon infection with *Leishmania* parasites, it is diminished and a prolonged duration of Leishmaniasis indicated by prolonged footpad swelling is observed. However, after re-infection an efficient protection from the parasite is not affected anymore. This indicates that the Th1-type response is not completely abrogated by epidermal exposition to L-Ag. Furthermore, from the antibody populations detected after EAI, it was observed that the same EAI results in a more Th2-type like immune response which in turn might be responsible for the long lasting footpad swelling of mice pre-treated with L-Ag. Epidermal LCs were suggested as candidates to be responsible for the observed phenomenon. However, neither the definite allocation to a distinct cell type or compound nor the mechanism was unambiguously determined and both are still not known. To the contrary, the results obtained with EAI indicate that the gold bombardment is predominantly targeted to the epidermis leading to the suggestion of LCs as candidate, but minor fractions of antigen coated particles were also shown to be present in the dermal layers. Therefore, the observed phenomenon renders it entirely unclear whether it can be attributed to antigen passively reaching skin draining LNs and being presented by different DCs to elicit the observed reaction or to a subset of DCs taking up the antigen and migrating to said lymph nodes. This hypothesis was already shared by Kissenpfennig *et al.* (2005) who tracked the reaction of LC-depleted mice to certain antigens. As a consequence, it remained to be determined whether a certain subset of DCs or a certain amount a the antigen itself being directed to the dermis is responsible for the observed phenomenon.

In summary, whereas the prior art has provided insight into the induction of tolerance and in particular autoimmune diseases, allergies and graft versus host or host versus graft reaction after transplantation, so far no promising means have been developed to allow for the medicinally indicated generation of tolerance without simultaneously generating severe side-effects. Accordingly, the technical problem underlying the present invention was to provide such means and methods.

The solution to said technical problem is achieved by providing the embodiments characterized in the claims.

The present invention relates to the use of epidermal Langerhans cells (LCs) transfected with a nucleic acid molecule encoding, in expressible form, an antigen or comprising an externally introduced antigen for the preparation of a pharmaceutical composition for inducing immune tolerance in a subject.

The term "antigen" refers to any kind of compound recognized by cells of the immune system as "foreign". As will be discussed in more detail below, sometimes antigens recognized as foreign are in fact antigens naturally present in the individual's own body. An antigen may be any type of molecule, a small molecular compound including small molecules (hapten) coupled to a carrier protein or a macromolecule such as a protein or nucleic acid molecule or fragments thereof as well as combinations of large molecules and small molecules coupled e.g. by chemical linkage. Said macromolecules or fragments thereof may comprise e.g. posttranslational modifications such as phosphorylation, methylation or glycosylation.
Two types of antigens are to be distinguished in accordance with this invention. Immunogens are antigens that provoke an immune response when introduced into the body. An immunogen comprises usually a macromolecule (protein, polysaccharide). Its ability to stimulate the immune reaction depends on its commonness to the host, molecular size, chemical composition and heterogeneity.
Allergens, forming the second group of antigens, are substances that cause allergic reactions. They can be ingested, inhaled, injected or come into contact with skin.
The group of tolerogens or autoantigens is to be distinguished from classical antigens capable of inducing an immune response. Tolerogens or autoantigens invoke a specific immune non-responsiveness. If their molecular form is changed, a tolerogen can become an immunogen.
Antigens can also be classified in order of their origins. Exogenous antigens are antigens that have entered the body from the outside, for example by inhalation, ingestion, or injection. By endocytosis or phagocytosis, these antigens are taken up into the antigen-presenting cells (APCs) and processed into fragments. APCs then present the fragments to T helper cells (CD4⁺) by the use of MHC class II molecules on their surface.
Endogenous antigens are antigens that have been generated within the cell, as a result of normal cell metabolism, or because of viral or intracellular bacterial infection. The fragments are then presented on the cell surface in the complex with class I histocompatibility molecules. If activated cytotoxic CD8⁺ T-cells (CTL) recognize them, the T-cells begin to secrete different toxins that cause the lysis or apoptosis of the infected cell. In order to keep the cytotoxic cells from killing cells just for presenting self-proteins, self-reactive T-cells are deleted from the repertoire as a result of central tolerance (also known as negative selection which occurs in the thymus). Only those CTL that do not react to self-peptides that are presented in the thymus in the context of MHC class I molecules are allowed to enter the bloodstream.
Due to the fact that nearly any molecule is able to serve as an antigen, the term "antigen" as used in connection with the present invention has to be interpreted in its broadest sense.
In the context of the present invention the term is not limited to one antigen. Especially in the case of transplantation antigens, it is envisaged to target and induce immune tolerance for more than one antigen, as several antigens differing from the recipient organism are expressed on the surface of donor cells.

The term "nucleic acid molecule" or "nucleic acid" as used in the context of this invention refers to a molecule such as a DNA or RNA molecule, which is doublestranded or single-stranded, as well as chimeras or molecules derived from the aforementioned by chemical modifications thereof. The molecule may be circular or linear. Linear molecules usually comprise only short DNA sequences such as oligonucleotides, that may or may not contain gene expression units, whereas circular molecules, in the form of plasmids may encode expression units, directing the expression of a protein encoded on the plasmid. The design and construction of such plasmids or DNA or RNA fragments is well known in the art. Applicable in accordance with the present invention is any suitable expression vector designed for expression in the desired target cell known in the art. Plasmids may also include DNA specific for certain microbes and therefore potentially acting as antigens. In general, any DNA molecule which is able to act as antigen either directly or indirectly by encoding an antigenic protein or peptide fragment may be used for practicing the invention. The DNA may also be modified in order to enhance immunogenicity, for example it may contain immunostimulatory CpG motifs or different modifications such as methylation. The DNA incorporated into a vector encodes normally proteins or fragments thereof, preferably derived from pathogens or allergens. However, vectors encoding fusion proteins are also envisaged. Generally, vectors can contain one or more origin of replication (ori) and inheritance systems for cloning or expression, one or more markers for selection in the host, e. g., antibiotic resistance, and one or more expression cassettes. Suitable origins of replication (ori) include, for example, the Col E1, the SV40 viral and the M 13 origins of replication.

DNA or a nucleic acid in expressible form comprises an expression vector suitable to direct expression of the desired protein or peptide in a host cell. The coding sequences inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in eukaryotic cells are well known to those skilled in the art. These elements comprise regulatory sequences ensuring the initiation of the transcription (e. g., translation initiation codon, promoters, enhancers, and/or insulators), internal ribosomal entry sites (IRES) (Owens et al., 2001) and optionally poly-A signals ensuring termination of transcription and stabilization of the transcript. Additional regulatory elements may include transcriptional as well as translational enhancers, and/or naturally-associated or heterologous promoter regions. Preferably, the polynucleotide of the invention is operatively linked to such expression control sequences allowing expression in eukaryotic cells. Furthermore, depending on the expression system used, leader sequences capable of directing the expressed polypeptide to a cellular compartment may be added to the coding sequence of the polynucleotide of the invention. Such leader sequences are well known in the art.

The term "immune tolerance" as used herein refers to the acquisition of non-reactivity towards particular antigens, which in this case are called tolerogen. It is naturally achieved by selection and killing of cells presenting self-antigens during the maturation process in the lymph node. A different way to induce immune tolerance is disclosed in the present invention, i. e. the modification of immune tolerance inducing LCs with the desired antigen or nucleic acid encoding said antigen resulting in the antigen becoming a tolerogen.

The term "subject" as used herein refers to any individual, preferably any mammal such as a human. The term in particular refers to an individual suffering from or being at risk to develop an autoimmune disease, graft vs. host reactions and host vs. graft reactions after receiving a transplant, allergies or any other kind of disease that involves inappropriate immune reactions.

So far the presentation of self-antigen by LCs has not been brought into connection with induction of immune tolerance, the treatment of autoimmune diseases, let alone the treatment of graft vs. host disease, host vs. graft disease or allergies, the latter involving reactions of the immune system to foreign antigens. Furthermore, the genetic manipulation of LCs in order to present non-self antigens for the treatment of allergies or to prevent rejection of a transplant has not been suggested in the prior art.

The experiments necessary for obtaining the results leading to the present invention included a method allowing for incorporation of Leishmania antigen into the epidermal compartment of the skin. For example, with a conventional "Gene gun" it is possible to expose epidermal cells to antigen, which is normally localized in the dermal or subcutaneous compartment. In 1992 Tang *et. al.* for the first time used a biolistic device for *in vivo* transfection by propelling gold microprojectiles coated with plasmid DNA into the skin of mice, thereby eliciting an immune response against the encoded antigen (Tang et al., 1992). The rationale to deliver vaccine antigens to the skin is to target antigen to epidermal LCs to facilitate antigen uptake and presentation and increase the magnitude of an induced immune response. Indeed, gene gun immunization with plasmid DNA has been demonstrated to be highly immunogenic and often superior to saline needle injection of plasmid DNA although much lower amounts of DNA are applied (Bennett, et al., 1999; Lodmell, et al., 2000; Weiss et al., 2000). However, the type of immune response elicited *via* gene gun immunization markedly differs from immune responses induced by needle application. Although gene gun immunization can induce strong CTL responses (Yoshida et al., 2000), which has been attributed to the intracellular delivery of antigen, the serological immune response is dominated by high levels of IgG1 and only low levels of IgG2a, indicating a Th2-biased reaction (Weiss et al. 2000; Feldquate et al., 1997; Oran et al., 2003). The differences in the observed immune reaction have been explained by the lower amounts of Th1 promoting immunostimulatory CpG motifs applied by gene gun immunization (Weiss et al., 2000; Barry & Johnston, 1997). Weiss et al. (2006) have shown that gene gun application can bias a Th1 immune reaction towards a serological Th2 type reaction in an antigen-independent manner (Weiss et al., 2002). Furthermore, it was shown that an efficient Th1-type response against intracellular pathogens can be modified by epidermal incorporation of Leishmania-antigen.
However, it was not possible to track down a specific subset of DCs or another component, e.g. the antigen itself, to act as inducing agent or to be responsible for the observed effects and modifications.
To determine said inducing agent, the experiments enclosed in the examples were carried out. Two scenarios were imaginable to cause the modified T-cell response as observed in Weiss et al. (2006) described above. Firstly, LCs which are known to transport antigen to skin draining LNs could account for the observed modification. On the other hand, the targeting of antigen with particle bombardment was intended to be directed to the epidermis, but a fraction was also found in dermal layers. Therefore, as second possibility, a passive transport of antigen from the dermis to the lymph nodes, where different cells capable to take it up and present it are located, could not be excluded.
OT-II cells are CD4-T-cells transgenic for a T-cell receptor recognizing an epitope on ovalbumin (OVA). LCs are positive for Langerin (CD207; LANG). Kissenpfennig et al. (2005) established a knock-in mouse strain that expressed the human diphtheria toxin (hDTR) under the control of the LANG promoter (B6.LANG-dHTR). Consequently, LCs can be depleted by application of DT.

The experiments carried out show measurable proliferation of OVA specific OT-II cells after ablation of LCs, reconstitution with OT-II cells and subsequent EAI with OVA antigen. On the other hand, in the presence of LCs the frequency of primed OT-II cells is much higher. Thus, LCs are the predominant cell population responsible for the transport of epidermally incorporated antigen.

However, as proliferation in the absence of LCs still occurs, even if to a lesser extent, an antigen contamination of dermal areas during gold bombardment cannot be excluded.
Another experiment demonstrates that LC ablation before epidermal antigen inoculation with EAI abrogates the chronic course of Leishmaniasis found in the wildtype B6 mouse. Since LCs were the only component of the system that was altered/absent in the experiment these results further support the findings discussed in context with T-cell activation in skin draining lymphnodes after ablation of LCs (above). Furthermore, the induction of chronic Leishmaiasis as reported by Weiss et al., 2006, can now be unambiguously attributed to the immune modulatory or tolerogenic potential and action of LCs.

The combined teachings of the prior art thus did not provide any clue how autoimmune diseases or transplant rejections could be successfully treated or inhibited with the help of epidermal LCs presenting self-antigens found in structures susceptible for autoimmune diseases or foreign antigens present on the cell surfaces of transplants.

Our data demonstrate for the first time that Langerin positive epidermal LCs (if loaded with antigen) are able to interfere with an antigen specific immune response. As a result, LCs might be used for the induction of immune tolerance in future.

The results of the research underlying the present invention characterized in the examples provide evidence that LCs are capable of mediating immune tolerance rather than other dendritic cells in skin draining LNs capable of presenting antigen which has passively reached these LNs. A treatment developed on the basis of these results leads to a medication or therapy rendering constant, lifelong medication dispensable, because modified LCs obtained from a subject suffering from allergies, an autoimmune disease or having received transplantation tissue can be reapplied without the need for further treatment. In case of subjects predisposed for autoimmune diseases or allergies, the therapy can be used in order to prevent the outbreak of the disease or allergy. As a matter of course the therapy has also to be applied prior to transplantation to prevent graft versus host or host versus graft reactions. Within the scope of the present invention, the method is aimed to predominantly target the epidermal layers of the skin, where tolerogenic LCs are located.

In a different embodiment the invention relates to the use of particles suitable for particle bombardment and an antigen or a nucleic acid molecule encoding an antigen for the preparation of a pharmaceutical composition for inducing immune tolerance in a subject.

The term "particle bombardment" refers to a technique well known in the art to introduce nucleic acid or protein molecules into cells. The application of gene transfer by particle bombardment was first described by Sanford *et al.,* (1987) and was shown to be an efficient method for transformation of many different organisms (Klein *et al.,* 1992). Mircoparticles, usually made from inert metals, are coated with a nucleic acid molecule and administered to the target tissue with high pressure, so that the nucleic acid-coated particles are fast enough to penetrate and transform living cells under *in vitro* and *in vivo* conditions. Any cell, tissue, or organ that can be made accessible for the application can be transformed. In the cell, particles can release their coating. In case of DNA, the respective molecules can then reach the nucleus and become integrated. Peptides are digested or processed to be presented as antigen on the surface of the respective cell.

In another embodiment the invention relates to the use of an antigen for the preparation of a composition for inducing immune tolerance in a subject, wherein said antigen is to be applied to the skin of a subject prior to particle bombardment of said skin, wherein said bombardment is suitable to introduce said antigen to a LC in the epidermis.

In the context of this embodiment of the present invention, the term "particle bombardment" also refers to a different approach. Whereas particle bombardment is usually carried out with antigen or nucleic acid coated particles, the present invention varies the established protocol. The particles themselves are preferably uncoated and directed to the skin of a subject, which has been contacted with the respective antigen or nucleic acid molecule prior to particle bombardment. Said contacting can be achieved, e. g. by application of a solution or a powder comprising the antigen or nucleic acid to the skin. Acceptable carriers can be aqueous solutions, possibly comprising additional substances.
Upon particle bombardment the particles transfer molecules in their trajectory into the targeted tissue. In case of DNA it may be incorporated and expressible DNA may be transcribed and translated to produce e. g. antigens. One major advantage of this novel method is that the aim, the transfection of cells, can be achieved with one step less in the procedure, saving time and being cost effective.
The method described above is suitable for any antigen, for which immune tolerance is intended. Proteins transferred into a cell may exert their antigenic effect shortly after introduction.

In a preferred embodiment the induction of immune tolerance in the subject is for preventing or treating autoimmune diseases, allergic reactions or graft vs. host disease or host versus graft disease after transplantation in a subject.

In another preferred embodiment of the invention the antigen is an allergen, an autoantigen or a transplantation antigen.

The term "transplantation antigen" as used in the context of the invention refers to an antigen transferred into the body of a subject upon transplantation. Although medical practitioners try to achieve the most possible accordance while assigning an organ to a receiving subject in need thereof, numerous structures differing from those of the host organism are still expressed on the surface of the graft cells, taken up by immune cells to be presented in connection with MHC class II (mouse) or HLA molecules (human) on their surface and to induce an immune response. All those differing structures are termed "transplantation antigens".

In another preferred embodiment of the invention the subject is of a mammalian species.

It is more preferred that the mammalian species is the human species.

In general, inert metals are used for particle bombardment, which themselves do not cause immune reactions in the host organism. Most prominent examples comprise gold or tungsten.

One of the newest technical systems used for the delivery of DNA-coated gold particles by helium gas shock wave is the Helios gene gun developed by Bio-Rad (Hercules, CA, USA). With the gene gun, a certain amount of DNA-coated particles is placed onto the inner surface of small Teflon tubes. After cutting the Teflon tube, up to 12 cartridges can be placed in the cartridge holder of the Helios gene gun at the same time. A high velocity helium gas stream causes a detachment of the particles from the surfaces. After that, the DNA-coated gold particles are fast enough to penetrate and transform living cells under *in vitro* and *in vivo* conditions. Any cell, tissue, or organ that can be made accessible for the gene gun can be transformed. No vacuum is necessary during the application. As described in the case of particle bombardment, supra, in the cell, particles can release their coating. In case of DNA, the respective molecules can then reach the nucleus and become integrated. Peptides - with the exception of super antigens - are digested and processed to be presented as antigen on the surface of the respective cell.

In another preferred embodiment the particle suitable for particle bombardment is a gold particle.
In another preferred embodiment the allergen is organic.

Organic compounds relate or belong to the class of chemical compounds having a carbon basis, the carbon atoms linked together by carbon-carbon bonds. The original definition of the term organic related to the source of chemical compounds, with organic compounds being those carbon-containing compounds obtained from plant or animal or microbial sources, whereas inorganic compounds were obtained from mineral sources. Organic compounds can be natural or synthetic. In these days allergies are often caused by artificially produced novel organic compounds, such as pesticides or plasticizers as well as low molecular weight pharmaceuticals, but also by certain microbial or plant proteins.

In a more preferred embodiment the organic allergen is a microbial allergen, an animal allergen or a plant allergen.

In another more preferred embodiment the organic allergen is selected from the group of low molecular weight pharmaceuticals, including analgetics and antibiotics, or chemical compounds such as ammonium persulfate.

In the context of the present invention "low molecular weight pharmaceuticals" are defined as compounds belonging to certain classes of pharmaceuticals commonly used for medical therapy. These include, but are not limited to analgetics, anesthetics, antibiotics and others.

The term "antibiotics" comprises any antibiotic that is, currently or in the future, used in human and veterinary clinical intervention. Commonly used antibiotics suitable for the treatment of infectious diseases in mammals and potentially causing allergies are e. g. beta-lactam antibiotics, doxycyclin or erythromycin.

Likewise, the term "analgetics" comprises any pharmaceutical that is, currently or in the future, used for analgesia in human and veterinary clinical intervention. Many commonly used analgetics are known to act as allergens, e. g acetylsalicaylic acid or diclofenac.
It is also preferred that the allergen is inorganic.

It is even more preferred that the inorganic allergen is selected from the group consisting of metal salts (nickel, chromium, cobalt, aluminum or platinum).

Inorganic compounds comprise chemical compounds that do not contain carbon as the principal element, that is, matter other than plant, microbial or animal. Generally, such compounds are derived from a non-living source, e. g. rocks, sand, and plastic or shells. Allergies against inorganic compound often refer to those against base metals, such as nickel, chromium or cobalt or salts thereof.

In a preferred embodiment of the invention the autoimmune disease comprises ongoing or chronic organ-specific autoimmune diseases such as multiple sclerosis, or systemic autoimmune diseases such as rheumatoid arthritis.

In another embodiment of the invention there is provided an *in vitro* method for preparing a tolerogenic composition for inducing immune tolerance in a subject, comprising contacting immature LCs obtained from a subject with an antigen or a nucleic acid molecule encoding an antigen to allow uptake of said antigen or said nucleic acid molecule by said LCs and culturing said cells to induce maturation, wherein said mature LCs are to be reapplied into the epidermis or a lymph node of the same subject.

Apart from particle bombardment suitable for cells on the surface of tissues different *in vitro* methods are known in the art to introduce nucleic acid molecules into cells. Commonly used methods comprise transfection using calcium chloride or lipofectin. As defined above for particle bombardment the nucleic acid molecule to be introduced can be linear or circular.

In a further preferred embodiment of the invention the tolerogenic composition for inducing immune tolerance is for preventing or treating autoimmune diseases, allergic reactions or graft vs. host disease or host vs. graft disease after transplantation in a subject.
In another embodiment of the invention there is disclosed a method of inducing immune tolerance in a subject comprising the step of introducing an antigen or a nucleic acid encoding said antigen into a LC in the epidermis of said subject. As described above, said introduction of an antigen or a nucleic acid can take place via particle bombardment or, in case of nucleic acids, by transfection of LCs.

In a preferred embodiment the immune tolerance is for preventing or treating autoimmune diseases, allergic reactions or graft vs. host disease or host vs. graft disease after transplantation in a subject.

In a more preferred embodiment the antigen or nucleic acid molecule encoding the antigen is coated to a particle and the antigen-coated particle is introduced into LCs via particle bombardment.

In another more preferred embodiment the antigen is applied to the skin of the subject followed by treatment of the skin with particle bombardment, preferably with uncoated particles, to introduce the antigen into the LCs.

In an even more preferred embodiment the particle is a gold particle.

In a most preferred embodiment the gold particle is accelerated to a velocity that enables penetration of the *stratum corneum* and is deposited in the epidermis of the subject. The penetration is achieved by applying a pressure of about 100 to 1000 psi.
The pressure to be applied varies depending on the species of the subject, the type of skin and the thickness thereof. The skilled person is able to choose the appropriate pressure upon respective information.

In another preferred embodiment of the present invention particles or particle bombardment is/are directed preferably exclusively to the epidermis to target LCs.

The present invention enables to induce immune tolerance by using epidermal Langerhans cells. Whereas particle bombardment as means to deliver antigen to LCs appears to be optimal to achieve the aim of the invention, other methods could be applied as well. Tape-stripping involves application and subsequent removing of an adhesive tape. Thereby, the upper layer of the epidermis is removed to make lower layers susceptible for antigen. After application of antigen it penetrates the epidermis and is taken up by epidermal LCs which then migrate to skin draining lymph nodes producing the desired effect.
Another means of delivery of antigen to LCs is realized with an system for transdermal delivery, such as an adhesive tape, covered with an organic agent or other penetration enhancer, e.g. DMSO, acetone or dibutylphthalate, and antigen. This organic "solvent" or other penetration enhancer enables for the transport of antigen into the epidermis where it is taken up by LCs, which migrate to skin draining lymph nodes.
Apart from the isolated antigen, also combined formulations are applicable. For example can the antigen be coupled to an antibody specifically recognizing LCs (e.g. α-Langerin, α-MHCII or α-CD1a). The resulting compound can be applied to the epidermis by either of the methods described above. Upon binding of the antibody to LCs, the antibody-antigen complex is taken up by the cell, the antigen is processed and presented on the cell surface.

Accordingly, the present invention also relates to the use of an antigen or nucleic acid molecule encoding an antigen for the preparation of a pharmaceutical composition for inducing immune tolerance in a subject, wherein the pharmaceutical composition is in the form of a transdermal delivery device.

In a preferred embodiment said antigen or nucleic acid molecule encoding an antigen is dissolved in an organic solvent or other penetration enhancer. Suitable solvents can be e.g. DMSO, acetone or dibutylphthalate.

In another preferred embodiment the transdermal delivery system is adhesive tape.

Adhesive tapes are commonly known in the art and the skilled person is aware of which kind of tape to use in connection with the present invention.

The invention also relates to a medical plaster, preferentially comprising a transdermal delivery system containing an antigen in an organic solvent or other penetration enhancer suitable to deliver said antigen to the LCs.

In a preferred embodiment the medical plaster is for inducing immune tolerance in a subject.

In another preferred embodiment the antigen is an autoantigen.

In a more preferred embodiment the transdermal delivery system is a tape, preferentially adapted for application to the human skin, wherein the tape is covered with an antigen and/or a solvent or penetration enhancer.

In a more preferred embodiment said transdermal delivery system contains said solvent. Suitable solvents can be e.g. DMSO, acetone or dibutylphthalate.

In an even more preferred embodiment said antigen is dissolved in said solvent or penetration enhancer.

The invention furthermore relates to the use of antigen or a nucleic acid encoding the antigen for the preparation of a pharmaceutical composition for inducing immune tolerance in a subject, wherein said composition is to be applied to the skin after tape-stripping.

The invention also relates to a kit comprising an adhesive tape or any other means suitable to be used for tape-stripping and an antigen in a pharmaceutically acceptable carrier.

Examples of suitable pharmaceutical carriers are well known in the art and include phosphate buffered saline solutions, water, emulsions, such as oil/water emulsions, various types of wetting agents, sterile solutions, etc. Compositions comprising such carriers can be formulated by well known conventional methods. These pharmaceutical compositions can be administered to the subject at a suitable dose. The dosage regimen will be determined by the attending physician and clinical factors.

In another preferred embodiment the antigen is coupled to an antibody specifically recognizing LCs. Exemplary antibodies recognizing LCs are α-Langerin, α-MHCII class or α-CD1a.

The figures show:

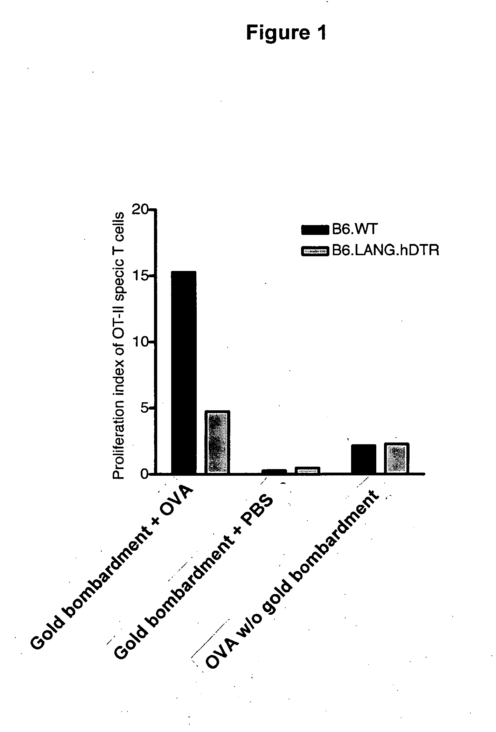

### Figure 1: OVA-specific T-cell proliferation is reduced after LC ablation.

500µg of OVA or PBS were applied abdominally on LC depleted B6.LANG.hDTR or B6.WT mice. 72 hours after gene gun application the proliferation of OVA-specific T-cells was measured in skin draining axillary lymph nodes. The proliferation index was calculated according to the formula: proliferating Vβ 5.1, 5.2 positive cells: non proliferating Vβ 5.1, 5.2 positive cells.

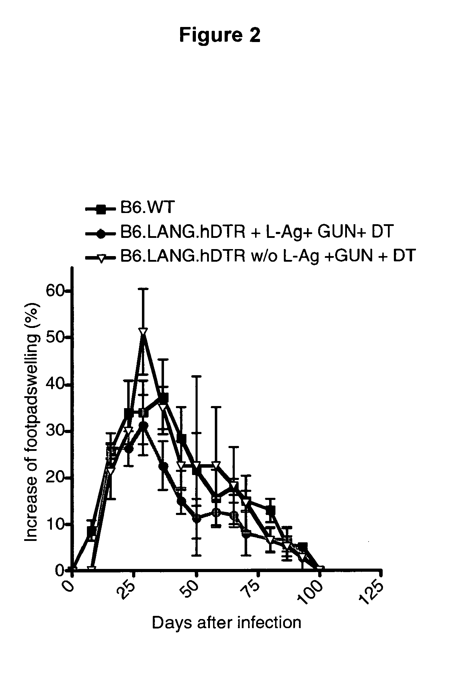

### Figure 2: Ablation of epidermal LCs before epidermal antigen incorporation abrogates the chronic course of experimental Leishmaniasis.

LC depleted B6.LANG.hDTR mice were treated with gold bombardment (GUN) plus either L-Ag (red circle) or PBS (black triangle). These mice and naïve B6.WT mice (black square) were infected with *L. major* parasites 20 days after epidermal antigen incorporation. Values represent the increase in footpad swelling compared to the uninfected hind footpad. Data are shown as mean ± SEM of individual footpad size. Data are representative of two independent experiments with 6 mice per group.

The examples illustrate the invention.

### Example 1: Materials and Methods

### Mice

Female B6.WT mice (aged 8-10 weeks) were purchased from Charles River (Sulzfeld, Germany). Ovalbumin (OVA) transgenic OT. II mice were maintained at the animal facility of the Bernhard Nocht Institute for Tropical Medicine or the University Hospital Hamburg, according to the guidelines for the care and use of experimental animals. B6.LANG-hDTR mice were kindly provided by A. Kissenpfennig

### Leishmania major parasites and the preparation of parasite antigen.

The cloned virulent *L*. *major* isolate (MHOM/IL/81/FEBNI) was propagated *in vitro* in blood agar cultures as previously described (Solbach et al., 1986). The virulence of the parasites was maintained by monthly passage in BALB/c mice. For the preparation of *L. major* parasites, stationary phase promastigotes from the third to seventh *in vitro* passage were harvested, washed four times, and re-suspended in PBS. Alternatively, the parasites were subjected to four cycles of rapid freezing and thawing to prepare L-Ag (Ritter et al., 2004).

### L. major infection and evaluation of the systemic course of disease

Mice were infected *via* subcutaneous injection of 3x10⁶ stationary-phase promastigotes into one of the hind footpads in a final volume of 50µl. The course of disease was monitored daily as described (Ritter et al., 2004). The parasite load in infected tissues was estimated using a Real-Time PCR assay as previously described in detail (Schulz et al., 2003).

### Flow cytometry analysis and proliferation assay

Multicolor flow cytometry was performed as described in detail (Ritter et al., 2004). The cells were electronically gated according to light-scatter properties and the expression of the TCR chains Vβ5.1,5.2 and CD4 were used to discriminate between donor and recipient cells. Proliferation of recipient cells was determined by labeling the cells with carboxyfluorescin succinimidyl ester (CFSE) as described (Siegmund et al., 2005). Data were collected using a FACS-Calibur^{™} flow cytometer (BD PharMingen) and analyzed with CellQuestPRO^{™} software (BD PharMingen).

### Epidermal antigen incorporation (EAI)

To verify whether EAI is sufficient to induce an OVA-specific T-cell activation in the skin draining LNs, we transferred intravenously 2x10⁷ CFSE labeled spleen cells from OT.II mice into B6.WT recipients as described previously (Weiss et al., 2006) or B6.LANG-hDTR. 24h after cell transfer 10µl OVA [50mg/ml in H₂O] were painted on the upper side of the hind footpad. After evaporation of liquid, the respective skin area was bombarded with 1.6µm gold particles using a Helios^{™} Gene gun (Bio-Rad, Hercules, CA) at a helium discharge pressure of 400psi. Similarly, 10µl of L-Ag [0.651µg/µl in PBS] was applied for EAI prior to infection studies with *L. major* promastigotes.

### Example 2: Langerhans cell-dependent proliferation of OVA-specific T-cells

LCs are positive for Langerin (LANG). Kissenpfennig et al. established a knock in mouse strain that expresses the human diphtheria toxin receptor (hDTR) under the control of the LANG promotor (B6.LANG-hDTR) (Kissenfpennig et al. 2005). Consequently, LANG-positive LCs can be depleted by DT application. For LC ablation, 1µg of DT (Sigma-Aldrich, Vienna, Austria) was injected intraperitoneally (i.p.) in 100µL pyrogen-free PBS two days before epidermal antigen incorporation.

B6.LANG-hDTR mice were reconstituted with CFSE labeled OT-II spleen cells after LC ablation. 24 hours later, recombinant ovalbumin (OVA) was inoculated intradermally as described previously (compare Weiss et al., 2006). The proliferation of OVA specific OT-II cells was determined by FACS analysis (figure 1). Our data demonstrate that in the absence of epidermal LCs proliferation of OVA-specific T cells is measurable in skin draining LNs. However, in the presence of LCs the frequency of primed OT-II cells is much higher (factor 3).
This result indicates that epidermal antigen inoculation by gold bombardment is sufficient to introduce antigen predominantly into the epidermal compartment (compare step 1, figure 1). Furthermore, LCs are the predominant cell population responsible for the transport of epidermally incorporated antigen.
Our results finally demonstrate that antigen contamination of dermal areas cannot be excluded. As shown, antigen incorporated into the dermis could be passively transferred to skin draining lymph nodes and mediate a T-cell response.

### Example 3: Langerhans cell ablation before epidermal antigen application

We carried out the experiments published by Weiss et al. with B6.LANG.hDTR mice (Weiss et al., 2006). LCs were depleted before epidermal inoculation of L-Ag. After 14 or 20 days mice were infected with 3x10⁶ promastigote *Leishmania* parasites. The increase of footpad swelling was measured weekly. LC ablation before epidermal antigen inoculation abrogated the chronic course of Leishmaniasis (figure 2).
This result indicates that in the model of experimental Leishmaniasis, LCs represent epidermal dendritic cells with immune-modulatory capacity (compare step 2, figure 2).

### References

Alexander, J. & Bryson, K. T helper (h)1/Th2 and Leishmania: paradox rather than paradigm. Immunol Lett 2005, 99(1), 17-23.
Anjuere, F., Martin, P., Ferrero, I. et al. Definition of dendritic cell subpopulations present in the spleen, Peyer's patches, lymph nodes, and skin of the mouse. Blood 1999, 93(2), 590-598.
Barry, M.A. & Johnston, S.A. Biological features of genetic immunization. Vaccine 1997, 15(8), 788-791.
Belkaid, Y., Von Stebut, E., Mendez, S. et al. CD8+ T cells are required for primary immunity in C57BL/6 mice following low-dose, intradermal challenge with Leishmania major. J Immunol 2002, 168(8), 3992-4000.
Bennett, A.M., Phillpotts, R.J., Perkins, S.D., Jacobs, S.C. & Williamson, E.D. Gene gun mediated vaccination is superior to manual delivery for immunisation with DNA vaccines expressing protective antigens from Yersinia pestis or Venezuelan Equine Encephalitis virus. Vaccine 1999, 18(7-8), 588-596.
Feltquate, D.M., Heaney, S., Webster, R.G. & Robinson, H.L. Different T helper cell types and antibody isotypes generated by saline and gene gun DNA immunization. J Immunol 1997, 158(5), 2278-2284.
Gilliam, A. C., Kremer, I. B., Yoshida, Y., Stevens, S. R., Tootell, E., Teunissen, M. B., Hammerberg, C. Cooper, K. D. The human hair follicle: a reservoir of CD40+ B7-deficient Langerhans cells that repopulate epidermis after UVB exposure. J Invest Dermatol 1998, 110: 422.
Ginhoux, F., Tacke, F., Angeli, V., Bogunovic, M., Loubeau, M., Dai, X. M., Stanley, E. R., Randolph, G. J., Merad, M. Langerhans cells arise form monocytes in vivo. Nat Immunol 2006, 7: 265.
Glaser, R., Harder, J., Lange, H., Bartels, J., Christophers, E. & Schroder, J.M. Antimicrobial psoriasin (S100A7) protects human skin from Escherichia coli infection. Nat Immunol 2005, 6(1), 57-64.
Gleich GJ. Mechanisms of eosinophil-associated inflammation. J Allergy Clin Immunol 2000; 105: 651-663.
Hauber H-P, Bergeron C, and Hamid Q. IL-9 in allergic inflammation. Int Arch Allergy Immunol 2004; 134: 79-87.
Itano, A.A. & Jenkins, M.K. Antigen presentation to naive CD4 T cells in the lymph node. Nat Immunol 2003, 4(8), 733-739.
Jackola DR, Pierson-Mullany LK, Liebeler CL et al. Variable binding affinities for allergen suggest a "selective competition" among immunoglobulins in atopic and nonatopic humans. Mol Immunol 2002; 39: 367-377.
Johansson SGO, Hourihane JOB, Bousquet J et al. A revised nomenclature for allergy. Allergy 2001; 56: 813-824.
Kamath, A. T., Henri, S., Battye, F., Tough, D. F., Shortman, K. Developmental kinetics and lifespan of dendritic cells in mouse lymphoid organs. Blood 2002, 100:1734.
Kaplan, D.H., Jenison, M.C., Saeland, S., Shlomchik, W.D. & Shlomchik, M.J. Epidermal langerhans cell-deficient mice develop enhanced contact hypersensitivity. Immunity 2005, 23(6), 611-620.
Kissenpfennig, A., Henri, S., Dubois, B. et al. Dynamics and function of Langerhans cells in vivo: dermal dendritic cells colonize lymph node areas distinct from slower migrating Langerhans cells. Immunity 2005, 22(5), 643-654.
Kissenpfennig A, Malissen B. Langerhans cells - revisiting the paradigm using genetically engineered mice. Trends Immunol. 2006 Mar; 27(3):132-9.
Kretschmer, K., Apostolou, I., Hawiger, D., Khazaie, K., Nussenzweig, M.C. & von Boehmer, H. Inducing and expanding regulatory T cell populations by foreign antigen. Nat Immunol 2005, 6(12), 1219-1227.
Lodmell, D.L., Ray, N.B., Ulrich, J.T. & Ewalt, L.C. DNA vaccination of mice against rabies virus: effects of the route of vaccination and the adjuvant monophosphoryl lipid A (MPL). Vaccine 2000, 18(11-12), 1059-1066.
Mayerova, D., Parke, E.A., Bursch, L.S., Odumade, O.A. & Hogquist, K.A. Langerhans cells activate naive self-antigen-specific CD8 T cells in the steady state. Immunity 2004, 21(3), 391-400.
Mazzarella G, Bianco A, Catena E et al. Th1/Th2 lymphocyte polarization in asthma. Allergy 2000; 55: S6-9.
Misslitz, A.C., Bonhagen, K., Harbecke, D., Lippuner, C., Kamradt, T. & Aebischer, T. Two waves of antigen-containing dendritic cells in vivo in experimental Leishmania major infection. Eur J Immunol 2004, 34(3), 715-725.
Moll, H., Fuchs, H., Blank, C. & Rollinghoff, M. Langerhans cells transport Leishmania major from the infected skin to the draining lymph node for presentation to antigen-specific T cells. Eur J Immunol 1993, 23(7), 1595-1601.
Ohl, L., Mohaupt, M., Czeloth, N. Hintzen, G., Kiafard, Z., Zwirner, J., Blankenstein, T., Henning, G., Forster, R. CCR7 governs skin dentritic cell migration under inflammatory and steady-state conditions. Immunity 2004, 21:279.
Oran, A.E. & Robinson, H.L. DNA vaccines, combining form of antigen and method of delivery to raise a spectrum of IFN-gamma and IL-4-producing CD4+ and CD8+ T cells. J Immunol 2003, 171(4), 1999-2005.
Owens G. C., Chappell S. A., Mauro V. P., Edelman G. M. (2001). Identification of two short internal ribosome entry sites selected from libraries of random oligonucleotides. Proc Natl Acad Sci USA 13(98), 1471-1476Proc Natl Acad Sci U S A. 2001 Feb 13;98(4):1471-6
Pearson, R.D., Wheeler, D.A., Harrison, L.H. & Kay, H.D. The immunobiology of leishmaniasis. Rev Infect Dis 1983, 5(5), 907-927.
Probst, H.C., Lagnel, J., Kollias, G. & van den Broek, M. Inducible transgenic mice reveal resting dendritic cells as potent inducers of CD8+ T cell tolerance. Immunity 2003, 18(5), 713-720.
Ritter, U., Wiede, F., Mielenz, D., Kiafard, Z., Zwirner, J., Korner, H. Analysis of CCR7 expression on murine bone marrow-derived and spleen dendritic cells. J Leucoc Biol 2004, 76: 472.
Ritter, U., Moll, H., Laskay, T. et al. Differential expression of chemokines in patients with localized and diffuse cutaneous American leishmaniasis. J Infect Dis 1996, 173(3), 699-709.
Ritter, U., Meissner, A., Scheidig, C. & Korner, H. CD8 alpha- and Langerin-negative dendritic cells, but not Langerhans cells, act as principal antigen-presenting cells in leishmaniasis. Eur J Immunol 2004, 34(6), 1542-1550.
Ritter, U. & Osterloh, A. A new view on cutaneous dendritic cell subsets in experimental leishmaniasis. Med Microbiol Immunol (Berl) 2006.
Robinson DS, Hamid Q, Ying S et al. Predominant Th2-like bronchoalveolar T-lymphocyte population in atopic asthma. N Engl J Med 1992; 326: 298-304. Shortman, K. Burnet oration: dendritic cells: multiple subtypes, multiple origins, multiple functions. Immunol Cell Biol 2000, 78: 161
Sacks, D. & Noben-Trauth, N. The immunology of susceptibility and resistance to Leishmania major in mice. Nat Rev Immunol 2002, 2(11), 845-858.
Schulz, A., Mellenthin, K., Schonian, G., Fleischer, B. & Drosten, C. Detection, differentiation, and quantitation of pathogenic leishmania organisms by a fluorescence resonance energy transfer-based real-time PCR assay. J Clin Microbiol 2003, 41(4), 1529-1535.
Siegmund, K., Feuerer, M., Siewert, C. et al. Migration matters: regulatory T-cell compartmentalization determines suppressive activity in vivo. Blood 2005, 106(9), 3097-3104.
Solbach, W., Forberg, K., Kammerer, E., Bogdan, C. & Rollinghoff, M. Suppressive effect of cyclosporin A on the development of Leishmania tropica-induced lesions in genetically susceptible BALB/c mice. J Immunol 1986, 137(2), 702-707.
Steinman, R.M. & Nussenzweig, M.C. Avoiding horror autotoxicus: the importance of dendritic cells in peripheral T cell tolerance. Proc Natl Acad Sci U S A 2002, 99(1), 351-358.
Stoitzner, P., Tripp, C.H., Douillard, P., Saeland, S. & Romani, N. Migratory Langerhans cells in mouse lymph nodes in steady state and inflammation. J Invest Dermatol 2005, 125(1), 116-125.
Strobl, H. Functional involvement of E-cadherin in TGF-beta 1-induced cell cluster formation of in vitro developing human Langerhans-type dendritic cells. J Immunol 2000, 165: 1381.
Tang, D.C., DeVit, M. & Johnston, S.A. Genetic immunization is a simple method for eliciting an immune response. Nature 1992, 356(6365), 152-154.
Valladeau, J., Clair-Moninot, V., Dezutter-Dambuyant, C. et al. Identification of mouse langerin/CD207 in Langerhans cells and some dendritic cells of lymphoid tissues. J Immunol 2002, 168(2), 782-792.
Weiss, R., Scheiblhofer, S., Freund, J., Ferreira, F., Livey, I. & Thalhamer, J. Gene gun bombardment with gold particles displays a particular Th2-promoting signal that over-rules the Th1-inducing effect of immunostimulatory CpG motifs in DNA vaccines. Vaccine 2002, 20(25-26), 3148-3154.
Weiss, R., Scheiblhofer, S., Thalhamer, J., Bickert, T., Richardt, U., Fleischer, B. & Ritter, U. Epidermal inoculation of Leishmania-antigen by gold bombardment results in a chronic form of leishmaniasis. Vaccine 2006;
Weiss, R., Leitner, W.W., Scheiblhofer, S. et al. Genetic vaccination against malaria infection by intradermal and epidermal injections of a plasmid containing the gene encoding the Plasmodium berghei circumsporozoite protein. Infect Immun 2000, 68(10), 5914-5919.
Wilhelm, P., Ritter, U., Labbow, S. et al. Rapidly fatal leishmaniasis in resistant C57BU6 mice lacking TNF. J Immunol 2001, 166(6), 4012-4019.
Wills-Karp M. Interleukin-13 in asthma pathogenesis. Immunol Rev 2004; 202: 175-190.
Wilson, N.S., El-Sukkari, D., Belz, G.T. et al. Most lymphoid organ dendritic cell types are phenotypically and functionally immature. Blood 2003, 102(6), 2187-2194.
Woods, G.M., Chen, Y.P., Dewar, A.L., Doherty, K.V., Toh, B.H. & Muller, H.K. Prevention of autoimmunity by induction of cutaneous tolerance. Cell Immunol 2001, 207(1), 1-5.
Yoshida, A., Nagata, T., Uchijima, M., Higashi, T. & Koide, Y. Advantage of gene gun-mediated over intramuscular inoculation of plasmid DNA vaccine in reproducible induction of specific immune responses. Vaccine 2000, 18(17), 1725-1729.

## Claims

1. Use of epidermal Langerhans cells transfected with a nucleic acid molecule encoding, in expressible form, an antigen or comprising an externally introduced antigen for the preparation of a pharmaceutical composition for inducing immune tolerance in a subject.

2. Use of particles suitable for particle bombardment and an antigen or a nucleic acid molecule encoding an antigen for the preparation of a pharmaceutical composition for inducing immune tolerance in a subject.

3. Use of an antigen for the preparation of a composition for inducing immune tolerance in a subject, wherein said antigen is to be applied to the skin of a subject prior to particle bombardment of said skin, wherein said bombardment is suitable to introduce said antigen to a Langerhans cell into the epidermis.

4. The use according to any one of claims 1 to 3, wherein the induction of immune tolerance in said subject is for preventing or treating autoimmune diseases, allergic reactions, graft vs. host disease or host vs. graft disease after transplantation in a subject.

5. The use according to any one of claims 1 to 4, wherein said antigen is an allergen, an autoantigen or a transplantation antigen.

6. The use according to any one of claims 1 to 5, wherein said subject is of a mammalian species.

7. The use according to claim 6, wherein said mammalian species is the human species.

8. The use of any one of claims 1 to 7, wherein said particle suitable for particle bombardment is a gold particle.

9. The use according to claim 5, wherein said allergen is organic.

10. The use according to claim 9, wherein said organic allergen is derived from plants, animals, or microbes.

11. The use according to claim 9, wherein said organic allergen is selected from the group of low molecular weight pharmaceuticals including analgetics and antibiotics.

12. The use according to claim 5, wherein said allergen is inorganic.

13. The use according to claim 12, wherein the inorganic allergen is selected from the group of metals comprising Aluminium, Chromium, Cobalt, Nickel and Platinum and their salts, or low molecular weight compounds such as ammonium persulfate.

14. The use according to any one of claims 4 to 8, wherein said autoimmune disease comprises ongoing organ-specific autoimmune diseases such as multiple sclerosis or systemic autoimmune diseases such as rheumatoid arthritis.

15. An *in vitro* method for preparing a tolerogenic composition for inducing immune tolerance in a subject, comprising contacting immature Langerhans cells obtained from a subject with an antigen or a nucleic acid molecule encoding an antigen to allow uptake of said antigen or said nucleic acid molecule by said Langerhans cells and culturing said cells to induce maturation, wherein said mature LCs are to be reapplied into the epidermis or a lymph node of the same subject.

16. The method according to claim 15 wherein said tolerogenic composition for inducing immune tolerance is for preventing or treating autoimmune diseases, allergic reactions, graft vs. host disease or host vs. graft disease after transplantation in a subject.

17. A method of inducing immune tolerance in a subject comprising the step of introducing an antigen or a nucleic acid encoding said antigen into a Langerhans cell in the epidermis of said subject.

18. The method according to claim 17, wherein said immune tolerance is for preventing or treating autoimmune diseases, allergic reactions, graft vs. host disease or host vs. graft disease after transplantation in a subject.

19. The method of claim 17 or 18, wherein said antigen or said nucleic acid molecule encoding said antigen is coated to a particle and said antigen-coated particle is introduced into said Langerhans cell via particle bombardment.

20. The method of claim 17 or 18, wherein said antigen is applied to the skin, wherein said skin is treated by particle bombardment with preferably uncoated particles to introduce said antigen into said Langerhans cells.

21. The method of claims 19 or 20, wherein said particle is a gold particle.

22. The method of claim 21, wherein said gold particle is accelerated to a velocity that enables penetration of the stratum corneum and is deposited in the epidermis of said subject and wherein said penetration is achieved by applying a pressure of about 100 to 500 psi.
